# FASCICULE DE BREVET EUROPEEN

(11) **EP 2 771 344 B1**
(45) Date de publication et mention de la délivrance du brevet: **13.04.2016**
(21) Numéro de dépôt: 12790609.7
(22) Date de dépôt: 23.10.2012
(51) Int. Cl.: C07D 487/04

(54) **ANALOGUES DE NUCLEOSIDES POUR LE TRAITEMENT D'UNE INFECTION VIRALE, ET METHODE D'EVALUATION DE LA SENSIBILITE AUDIT TRAITEMENT**
NUKLEOSID-ANALOGA ZUR BEHANDLUNG EINER VIRUSINFEKTION UND VERFAHREN ZUR AUSWERTUNG DER EMPFINDLICHKEIT GEGEN DIESE BEHANDLUNG
NUCLEOSIDE ANALOGUES FOR TREATING A VIRAL INFECTION, AND METHOD FOR EVALUATING THE SENSITIVITY TO SAID TREATMENT

(30) Priorité: 24.10.2011 FR 1159622
(43) Date de publication de la demande: 03.09.2014
(73) Titulaire: Université Pierre et Marie Curie (Paris 6), 75005 Paris (FR)
(72) Inventeur: CALVEZ, Vincent, F-75005 Paris (FR); MARCELIN, Anne-Geneviève, F-75005 Paris (FR); SOULIE, Cathia, F-75012 Paris (FR); ETHEVE-QUELQUEJEU, Mélanie, F-94600 Choisy-Le-Roi (FR); SOLLOGOUB, Matthieu, F-75018 Paris (FR)
(74) Mandataire: Chajmowicz, Marion
(86) Numéro de dépôt international: PCT/FR2012/052433
(87) Numéro de publication internationale: WO 2013/060980

(56) Documents cités:
- EP-A2- 0 462 621
- WO-A2-2005/084653
- US-A1- 2003 008 841
- XUE-FENG ZHU AND CO: ""Novel synthetic approach to multibenzoylated nucleosides"", SYNTHETIC COMMUNICATIONS, vol. 38, 1 janvier 2008 (2008-01-01), pages 1346-1354, XP002667614,
- TAKESHI ENDO AND CO: ""Oxidation of N,N-dialkyl-2',3',5'-tri-O-acetyladenosine s with Ruthenium tetraoxide and a novel selective N-Monodealkylation sequence"", JOURNAL OF ORGANIC CHEMISTRY, vol. 44, no. 21, 1 janvier 1979 (1979-01-01), pages 3652-3656, XP002667615,
- QING DAI AND CO: ""Efficient chemical synthesis of AppDNA by adenylation of immobilized DNA-5'-monophosphate"", ORGANIC LETTERS, vol. 11, no. 5, 1 janvier 2009 (2009-01-01), pages 1067-1070, XP002667616,

## Description

La présente invention décrit des analogues de nucléosides et leur utilisation pour le traitement d'une infection virale, en particulier une infection par le Virus de l'Immunodéficience acquise Humaine (VIH).

### Arrière-plan de l'invention

Les médicaments proposés dans le traitement du VIH peuvent être répartis en six classes : les inhibiteurs de la transcriptase inverse nucléosidiques (nucléotidiques) [N(t)RTIs], les inhibiteurs de la transcriptase inverse non nucléosidiques [NNRTIs], les inhibiteurs de protéase [Pls], les inhibiteurs d'intégrase [INIs], les antagonistes de CCR5, et les inhibiteurs de fusion.

L'introduction d'une thérapie antirétrovirale hautement active comme traitement du VIH a permis de réduire de façon significative la mortalité et la morbidité pour les patients atteints du VIH dans le monde. Les traitements initiaux recommandés pour l'infection par le VIH incluent au moins deux médicaments connus pour appartenir à la classe des inhibiteurs de transcriptase inverse nucléosidiques.

Cependant, la plupart des composés utilisés posent des problèmes de toxicité, et tous rencontrent des problèmes de résistance. C'est pourquoi on recherche une diminution des effets secondaires et une amélioration des dosages (par exemple administration, une fois par jour, de pilules de combinaison à dose fixe), lesquelles permettraient une compliance plus élevée des patients, limiteraient l'émergence de variants résistants, et ainsi amélioreraient la longévité et la qualité de vie des patients.

Il existe donc un besoin de nouvelles molécules antivirales qui soient à la fois bien tolérées et efficaces et aussi ayant de nouveaux mécanismes d'action.

Dans ce cadre, les inventeurs ont démontré qu'une sous-famille d'analogues benzoylés de nucléosides présente de façon surprenante une activité antivirale intéressante à la fois sur le virus VIH natif et sur les variants résistants, tout en étant très peu toxique.

### Résumé de l'invention

La présente invention concerne des composés de formule (I) pour leur utilisation dans le traitement d'une infection virale, en particulier une infection par un virus VIH.

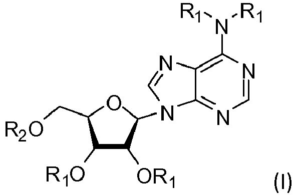

La présente invention concerne également une composition pharmaceutique comprenant au moins un composé de formule (I) et un support pharmaceutiquement acceptable.

La présente invention fournit en outre une méthode *in vitro* pour évaluer si un patient infecté par un virus VIH, notamment VIH-1, serait sensible à une thérapie avec un composé tel que défini ici, ladite méthode comprenant la recherche d'une mutation dans le codon 215 de la transcriptase inverse du virus, une substitution de la thréonine sauvage, en tyrosine, étant indicatrice d'une plus grande sensibilité audit composé, par rapport au virus sauvage.

### Description des figures

La Figure 1 est un graphe montrant la mesure de l'IC50 (concentration inhibitrice à 50%) de l'adénosine tétrabenzoylée, comparée avec celle du TDF (Ténofovir). Les carrés correspondent au Ténofovir et les losanges à la tétrabenzoyladénosine (N⁶,N⁶,O^{2'},O^{3'}-tétrabenzoyladénosine de formule **A** telle que décrite ci-dessous).
La Figure 2 est un graphe montrant le nombre de copies d'ADN viral pour 150 000 cellules MT2 infectées, en fonction du temps (T= témoin, AZT= zidovudine).
La Figure 3 est un graphe montrant le nombre de copies d'ARN viral par ml de cellules MT2 infectées, en fonction du temps (T= témoin, AZT= zidovudine).

### Description détaillée de l'invention

La présente invention concerne un composé de formule (I) 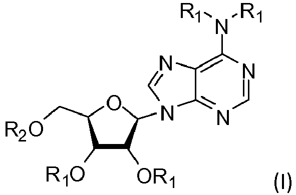 dans laquelle
chaque R1 est indépendamment choisi parmi un groupement benzyle et un groupement benzoyle (Bz), éventuellement substitué par un ou plusieurs substituants choisis parmi un groupement nitro (NO₂) un groupement alkyle, et un atome d'halogène ;
R2 est choisi parmi un atome d'hydrogène, un groupement acyle, un monophosphate, diphosphate ou triphosphate, ou un dérivé phosphate stabilisé,
un isomère, tautomère ou énantiomère de celui-ci, une prodrogue ou un sel pharmaceutiquement acceptable de celui-ci, ou un mélange de ceux-ci, pour son utilisation dans le traitement d'une infection virale.

Le virus peut être par exemple le VIH (virus de l'immunodéficience humaine), le virus de l'hépatite C (HCV), le virus de l'hépatite B ou le virus de l'herpès.

Les composés sont particulièrement utiles pour traiter une infection virale chez des patients infectés par une souche virale porteuse d'une mutation leur conférant une résistance aux traitements actuels, par exemple une résistance aux inhibiteurs de transcriptase inverse nucléosidiques ou non-nucléosidiques, ou aux inhibiteurs de l'intégrase ou de la protéase, en particulier en ce qui concerne le VIH.

En particulier, l'infection virale est le VIH.

Tous les génotypes sont compris, notamment les groupes VIH-1, VIH-2, VIH-O.

Les composés sont utiles pour traiter une infection chez n'importe quel patient. Les composés sont plus particulièrement efficaces pour lutter contre une infection par un virus VIH, notamment VIH-1, porteur de la mutation T215Y.

De préférence un, de préférence deux, de préférence trois et encore préférentiellement les quatre groupements R1 sont des groupements benzoyles.

De préférence, R2 est choisi parmi un atome d'hydrogène, un groupe 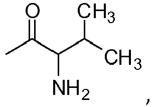 et un dérivé phosphate stabilisé choisi parmi un groupe 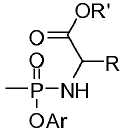 et un groupe 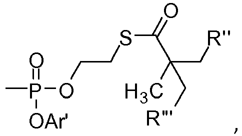 dans lesquels Ar et Ar' sont des groupements phényles ou naphtyles éventuellement substitués par un ou plusieurs atomes d'halogène, et R, R', R" et R"' sont indépendamment choisis parmi un atome d'hydrogène, un groupement hydroxy (OH), un alkyle, un phényle et un groupement amine NRaRb où Ra et Rb sont choisis parmi les atomes d'hydrogènes, les alkyles et les halogénoalkyles.

De façon hautement préférée, R2 est un atome d'hydrogène.

Par « groupement alkyle », on désigne dans la présente invention un groupement hydrocarboné, linéaire, ramifié ou cyclique, comprenant 1 à 8 atomes de carbone, éventuellement substitué par au moins un substituant choisi parmi les atomes d'halogène, les groupes hydroxy (OH) et amino (NH₂). A titre d'exemples, on peut citer les groupements méthyle, éthyle, propyle, isopropyle, n-butyle, sec-butyle, pentyle, hexyle, cyclohexyle, heptyle, et octyle.

Par « groupement acyle », on désigne dans la présente invention un groupe alkyle relié au reste de la molécule par l'intermédiaire d'un C=O.

Par « dérivé phosphate stabilisé », on désigne dans la présente invention un groupement dérivé d'un groupement phosphate et permettant de former une prodrogue nucléotidique stabilisée.

Par « atome d'halogène », on désigne dans la présente invention un atome de chlore, de brome, d'iode ou de fluor.

Par « halogénoalkyle », on désigne dans la présente invention un groupement alkyle tel que défini ci-dessus, substitué par au moins un atome d'halogène. A titre d'exemple, on peut citer notamment le groupement trifluorométhyle.

Par « prodrogue » d'un composé de formule (I), on désigne dans la présente invention un composé qui est converti en composé monophosphorylé de formule (I) correspondant lors de son administration *in vivo*, ou qui a la même activité par lui-même. En particulier, on peut citer les sels pharmaceutiquement acceptables et certains dérivés en 5', en particuliers des dérivés hydrolysables, des composés actifs.

Par « sel pharmaceutiquement acceptable », on désigne dans la présente invention un sel d'un composé de formule (I) qui ne possède pas ou peu d'effet toxicologique non désiré. Il peut s'agir par exemple d'un sel d'addition acide comme un chlorhydrate ou un acétate ou d'un sel d'addition basique comme un sel de sodium ou de potassium.

Un composé de formule (I) peut être administré sous forme de prodrogue stabilisée par exemple pour améliorer son activité, sa biodisponibilité, sa stabilité ou pour altérer une quelconque propriété non désirée du composé de formule (I).

Parmi les composés de l'invention, le composé de formule **A** (N⁶,N⁶,O^{2'},O^{3'}-tétrabenzoyladénosine) est hautement préféré.

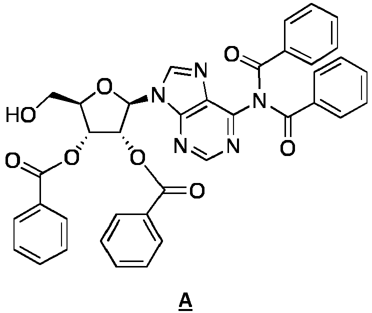

La N⁶,N⁶,O^{2'},O^{3'}-tétrabenzoyladénosine **A** peut être synthétisée comme décrit initialement par Khorana *et al.* (Lohrmann, R. ; Khorana, H. G. J. Am. Chem. Soc. 1964, 86, 4188-4194), ou par le procédé amélioré par Scott *e*t *al.* (Zhu, X.-F.; Scott, A. I. Synthetic Commun. 2008, 1346-1354) en utilisant le *tert*-butyldiméthylsilyle en tant que groupement protecteur temporaire en 5'-OH.

Les inventeurs ont démontré que la N⁶,N⁶,O^{2'},O^{3'}-tétrabenzoyladénosine a une activité antivirale (notamment anti-VIH) plus importante que le Ténofovir (TDF), qui est l'antirétroviral le plus utilisé pour le traitement des patients infectés par le VIH. Le bas niveau de toxicité cellulaire mesuré rend l'utilisation de ce composé particulièrement utile chez des patients infectés par le VIH. En outre, sans vouloir être liés à un quelconque mécanisme d'action, les inventeurs ont mis en évidence que ce composé agissait à une étape précoce de l'infection virale, à savoir avant l'intégration du virus dans l'ADN de la cellule. Plus particulièrement, les inventeurs ont montré une inhibition de la production de l'ADN proviral. Cette propriété des composés de l'invention est particulièrement avantageuse pour un traitement efficace, en amont de l'intégration cellulaire.

La présente invention concerne également une composition pharmaceutique comprenant au moins un composé de formule (I) et un support pharmaceutiquement acceptable.

Dans le contexte de l'invention, le terme « support pharmaceutiquement acceptable» désigne des substances telles que les excipients, les véhicules, les adjuvants, les tampons qui sont classiquement utilisés, en combinaison avec le(s) principe(s) actif(s), pour la préparation d'un médicament. Le choix de tels supports dépend essentiellement de la voie d'administration envisagée.

Dans un mode de réalisation particulier, les composés de l'invention peuvent être sous forme encapsulée, en étant par exemple introduits dans des microsphères ou des microcapsules qui sont des réservoirs constitués d'un coeur de principe actif entouré par une membrane de matériau enrobant. Les polymères formant le matériau enrobant peuvent être d'origine naturelle (gélatine, chitosane, ...), hémisynthétique (dérivés de la cellulose...) ou synthétique comme les copolymères des acides lactique et glycolique couramment utilisés. Les composés de l'invention peuvent être également encapsulés dans des nanoparticules, qui sont des systèmes colloïdaux dont la taille est comprise entre 10 et 1000 nm, à base de polymères biodégradables, ou de lipides capables de retenir une ou des molécules actives par séquestration et/ou adsorption.

La composition pharmaceutique selon l'invention comprend de préférence une quantité de composé selon l'invention comprise entre 5µg et 1000 mg, de préférence entre 1 et 500 mg, de préférence entre 5 et 100mg.

Le rapport entre les quantités en poids de composé selon l'invention et de support pharmaceutiquement acceptable est compris entre 5/95 et 95/5, de préférence entre 20/80 et 80/20.

Les composés de l'invention peuvent être les seuls principes actifs, ou ils peuvent être associés à d'autres principes actifs. La composition pharmaceutique selon l'invention peut ainsi également comprendre au moins un autre actif pharmaceutique, en particulier au moins un autre médicament utilisé pour le traitement de l'infection virale. En particulier, la composition selon l'invention peut comprendre également, ou être associée à, un ou plusieurs autres antirétroviraux, par exemple du Ténofovir. De manière générale, tout antirétroviral peut être associé, à savoir des inhibiteurs de la transcriptase inverse, notamment des inhibiteurs nucléosidiques ou nucléotidiques et non nucléosidiques, des inhibiteurs de la protéase, des inhibiteurs de l'intégrase, des inhibiteurs d'entrée (anti-CCR5), des inhibiteurs de fusion, et des inhibiteurs d'attachement au site de fixation du CD4. Parmi les antirétroviraux, on peut utiliser par exemple ceux actuellement commercialisés, tels que la lamivudine, l'emtricitabine, la zalcitabine, l'abacavir, la zidovudine, la didanosine, la stavudine, l'adéfovir, le ténofovir, l'éfavirenz, l'étravirine, la névirapine, la delavridine, la rilpivirine, l'amprénavir, le fosamprénavir, le tipranavir, le lopinavir, le ritonavir, l'indinavir, le saquinavir, le darunavir, l'atazanavir, le nelfinavir, le raltégravir, l'éviltegravir, le dolutégravir, l'enfuvirtide, le maraviroc.

Les composés ou compositions selon l'invention peuvent être administrés de différentes manières et sous différentes formes. Ainsi, ils peuvent être administrés de manière systémique, par voie orale, par inhalation ou par injection, comme par exemple par voie intraveineuse, intra-musculaire, sous-cutanée, trans-dermique, intra-artérielle, etc., les voies intraveineuse, intra-musculaire, sous-cutanée, orale et par inhalation étant préférées. Pour les injections, les composés sont généralement conditionnés sous forme de suspensions liquides, qui peuvent être injectées au moyen de seringues ou de perfusions, par exemple. A cet égard, les composés sont généralement dissous dans des solutions salines, physiologiques, isotoniques, tamponnées, etc., compatibles avec un usage pharmaceutique et connues de l'homme du métier. Ainsi, les compositions peuvent contenir un ou plusieurs agents ou véhicules choisis parmi les dispersants, solubilisants, stabilisants, conservateurs, etc. Des agents ou véhicules utilisables dans des formulations liquides et/ou injectables sont notamment la méthylcellulose, l'hydroxyméthylcellulose, la carboxyméthylcellulose, le polysorbate 80, le mannitol, la gélatine, le lactose, des huiles végétales, l'acacia, etc.

Les composés peuvent également être administrés sous forme de gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Il est entendu que le débit et/ou la dose injectée peuvent être adaptés par l'homme du métier en fonction du patient, de la pathologie concernée, du mode d'administration, etc. Typiquement, les composés sont administrés à des doses pouvant varier entre 0.1 µg et 100 mg/kg de poids corporel, plus généralement de 0,01 à 10 mg/kg, typiquement entre 0,1 et 10 mg/kg. Généralement on administre de 5µg et 1000 mg, de préférence entre 1 et 500 mg, de préférence entre 5 et 100mg par jour. En outre, des injections répétées peuvent être réalisées, le cas échéant. D'autre part, pour des traitements chroniques, des systèmes retard ou prolongés peuvent être utilisés.

La présente invention concerne également une méthode de traitement d'une infection virale, comprenant l'administration à un patient d'une quantité efficace d'au moins un composé de formule (I) et/ou d'une composition en contenant.

La présente invention a également pour objet l'utilisation d'au moins un composé tel que défini ci-dessus dans le cadre de la préparation d'une composition pharmaceutique destinée au traitement d'une infection virale.

Dans un objectif de médecine personnalisée, l'invention a en outre pour objet une méthode pour identifier les patients infectés par un virus VIH, en particulier VIH-1, qui seraient très sensibles à une thérapie avec une composition pharmaceutique décrite ici, ladite méthode comprenant la recherche d'une mutation dans le codon 215 de la transcriptase inverse, une substitution de la thréonine sauvage, en tyrosine, étant indicatrice d'une plus grande sensibilité à un composé de l'invention, par rapport à un virus sauvage. Par « patients très sensibles », on entend des patients pour lesquels une thérapie avec un composé de l'invention serait particulièrement avantageuse, étant entendu que les composés de l'invention restent aussi utiles pour traiter une infection par un virus sauvage ou porteur d'autres mutations.

La recherche de cette mutation peut être réalisée par toute technique standard, de génotypage, séquençage ou détection rapide par sondes (cf par exemple Ross, et al AIDS Research and Human Retroviruses, 1999, 15(14): 1287-1292; Mitsuya et al, J Virol. 2008, 82(21):10747-55).

Dans le contexte de l'invention, le terme « traitement » désigne le traitement préventif, curatif, palliatif, ainsi que la prise en charge des patients (réduction de la souffrance, amélioration de la durée de vie, ralentissement de la progression de la maladie, etc.). Le traitement peut en outre être réalisé en combinaison avec d'autres agents ou traitements chimiques ou physiques. Les composés selon l'invention sont de préférence conditionnés et administrés de façon combinée, séparée ou séquentielle par rapport à d'autres agents ou traitements thérapeutiques. Les traitements et médicaments de l'invention sont tout particulièrement destinés aux humains. Les patients susceptibles d'être traités par les composés et/ou compositions de l'invention peuvent notamment être une population de patients résistants aux antiviraux classiquement utilisés pour le traitement de l'infection virale, en particulier de patients (de préférence les patients infectés par le virus VIH) résistants aux inhibiteurs de transcriptase inverse nucléosidiques, non-nucléosidiques, ou aux inhibiteurs de l'intégrase ou de la protéase.

Les inhibiteurs nucléosidiques de la transcriptase inverse comprennent notamment les molécules suivantes : zidovudine, molécule également connue sous le nom AZT, lamivudine, emtricitabine, didanosine, stavudine, abacavir, zalcitabine, tenofovir, racivir, amdoxovir, apricitabine, elvucitabine.

Les inhibiteurs non-nucléosidiques de la transcriptase inverse comprennent notamment les molécules suivantes : efavirenz, nevirapine, étravirine, delavirdine, rilpivirine.

Les inhibiteurs de l'intégrase incluent l'elvitégravir et le dolutégravir.

Les inhibiteurs de la protéase incluent les molécules suivantes : amprenavir, tipranavir, indinavir, saquinavir, fosamprenavir, ritonavir, darunavir, atazanavir, nelfinavir.

Les composés selon l'invention peuvent être synthétisés de façon simple (en 3 étapes seulement) à partir, par exemple, d'adénosine commerciale.

Les trois étapes ont lieu sans étape de purification intermédiaire, il s'agit tout d'abord d'une étape de protection régiosélective de la position 5'- de l'adénosine en utilisant du chlorure de *tert*-butyldiméthylsilyle, suivie d'une protection de la fonction amine de la base et des deux autres hydroxyles en position 2'- et 3'- du ribose par du chlorure de benzoyle. L'étape terminale est la déprotection de l'hydroxyle en position 5'-, qui peut être réalisée par du fluorure de tétrabutylammonium ou de l'acide trifluoroacétique, de préférence de l'acide trifluoroacétique. Une étape de purification par colonne de chromatographie permet alors d'obtenir le composé d'intérêt.

D'autres aspects et avantages de la présente demande apparaîtront à la lecture des exemples qui suivent, qui doivent être considérés comme illustratifs et non limitatifs.

### Exemples

### Exemple 1 : Synthèse du composé N⁶,N⁶,O^{2'},O^{3'}-tétrabenzoyladénosine (composé A) selon l'invention

### - Exemple 1.1: Premier mode de réalisation

A une solution d'adénosine commerciale (540 mg, 2,0 mmol) et de pyridine (10 mL), du chlorure de *tert*-butyldiméthylsilyle (TBSCl) (396 mg, 2,6 mmol) est ajouté à 0°C. Après agitation pendant 2 h à 0°C, le mélange réactionnel est ramené à température ambiante et agité à nouveau pendant 7 h. La solution est ensuite refroidie à 0°C et du chlorure de benzoyle (1,12 mL, 9,6 mmol) est ajouté goutte à goutte. Après une nuit d'agitation à température ambiante, le mélange est dilué dans du CH₂Cl₂ (100 mL) et lavé avec une solution saturée de NaCl (40 mL). La phase aqueuse est extraite avec du CH₂Cl₂ (2 x 40 mL), les phases organiques sont rassemblées, séchées sur Na₂SO₄, filtrées et concentrées sous vide. Le brut réactionnel obtenu est dissous dans du THF (36 mL), et une solution aqueuse d'acide trifluoroacétique TFA (18 mL, TFA/H₂O 1:1) est additionnée lentement. Après 3h d'agitation à température ambiante, le mélange réactionnel est neutralisé avec une solution de NaHCO₃ (9,72 g dans 100 mL de H₂O) à 0°C et extraite avec du CH₂Cl₂ (4 x 50 mL). Les phases organiques sont rassemblées, séchées sur Na₂SO₄, et concentrées sous vide après filtration.

Le produit obtenu est purifié sur colonne de chromatographie (Eluant: cyclohexane/EtOAc = 4:1 à 1:1 puis CH₂Cl₂/CH₃OH 100:5) pour donner l'adénosine tétrabenzoylée sous forme d'un solide blanc. Rendement: 1.36 g (99%).

### - Exemple 1.2 : Second mode de réalisation

A une solution d'adénosine commerciale (3 g, 11,2 mmol) et de pyridine distillée (60 mL), du chlorure de *tert*-butyldiméthylsilyle (TBSCl) (2,2 g, 14,6 mmol) est ajouté à 0°C. Après agitation pendant 4 h à 0°C, le mélange réactionnel est ramené à température ambiante et agité à nouveau pendant une nuit. La pyridine est évaporée puis le mélange réactionnel est repris dans du CH₂Cl₂ (500 mL) et du MeOH (50 mL), et lavé avec une solution saturée de NaCl (500 mL) et de NaHCO₃ (500 mL). La phase aqueuse est extraite avec du CH₂Cl₂ (200 mL), les phases organiques sont rassemblées, séchées sur Na₂SO₄, filtrées et concentrées sous vide. Le solide est dissous dans 60 mL de pyridine, la solution est refroidie à 0°C et du chlorure de benzoyle (6,26 mL, 53,9 mmol) est ajouté goutte à goutte. Après une nuit d'agitation à température ambiante, le mélange est dilué dans du CH₂Cl₂ (300 mL) et lavé avec une solution saturée de NaCl (2x 150 mL). La phase aqueuse est extraite avec du CH₂Cl₂ (150 mL), les phases organiques sont rassemblées, séchées sur Na₂SO₄, filtrées et concentrées sous vide. Le brut réactionnel obtenu est dissous dans du THF (100 mL), et une solution aqueuse d'acide trifluoroacétique TFA (50 mL, TFA/H₂O 1:1) est additionnée lentement. Après une nuit d'agitation à température ambiante, le mélange réactionnel est neutralisé avec une solution de NaHCO₃ à 0°C et extrait avec du CH₂Cl₂ (2x 150 mL). Les phases organiques sont rassemblées, séchées sur Na₂SO₄, et concentrées sous vide après filtration.

Le produit obtenu est purifié sur colonne de chromatographie (Eluant: cyclohexane/EtOAc = 4:1 à 1:1 puis CH₂Cl₂/CH₃OH 100:5) pour donner l'adénosine tétrabenzoylée sous forme d'un solide blanc. Rendement: 5,96 g (77%).

### - Caractérisation du produit obtenu avec les 2 modes de réalisation

R*f*: 0.25 (cyclohexane/EtOAc = 2:1); NMR 1H: (CDCl₃) δ 8.70 (s, 1 H, H2 ou H8), 8.21 (s, 1 H, H2 ou H8), 8.05 (d, *J*=7.5 Hz, 2H, Bz), 7.33-7.86 (m, 18H, Bz), 6.36 (m,, 2H, H1', H2'), 6.06 (dd, *J* = 1,6, 5,2 Hz 1H, H3'), 5.56 (sl, 1H, OH), 4.64 (bs, 1H, H4'), 4.05 (m, 2H, 2 x H5') HRMS: calculé pour C38H29N5O8Na: 706.1914, trouvé 706.1917 [M+Na+].

### Exemple 2 : Tests antiviraux et de cytotoxicité

### Matériels et méthodes :

### Composés:

Le composé **A** (adénosine tétrabenzoylée, cf préparation à l'exemple 1) est dissous dans le DMSO (diméthylsulfoxyde) à 10 mM. Le Ténofovir commercial est dissous dans l'eau à 1 g/L.

### Cellules et virus:

Les cellules HeLa-P4 sont des cellules HeLa CD4 LTR-LacZ dans lesquelles l'expression de LacZ est induite par la protéine Tat trans-activatrice de VIH, rendant possible de la quantification précise de l'infectivité VIH-1 à partir d'un seul cycle de réplication. Des cellules HeLa-CD4 croissant de façon exponentielle à une densité de 1 x 10⁴ /mL sont placées dans des plaques 96 puits et infectées le jour suivant avec 3 ng d'antigène p24 de VIH en présence de différentes concentrations de composés.

### Test antiviral dans les cellules HeLa-P4

Les titres pour un simple cycle du virus ont été déterminés dans des cellules HeLa-P4. Les cellules ont été infectées, en duplicats, dans des plaques 96 puits, avec le clone VIH-1 pNL-4.3 (équivalent à 3 ng d'antigène p24). Les titres pour un simple cycle des virus ont été déterminés 48 heures après infection en quantifiant l'activité béta-galactosidase dans des lysats P4 par test colorimétrique (test CPRG) basé sur le clivage du chlorophénol red-beta-D-galactopyranoside (CPRG) par la béta-galactosidase. La concentration inhibitrice à 50% (IC50) a été déterminée comme la concentration de composé **A** fournissant 50% d'inhibition des niveaux de béta-galactosidase par rapport aux cellules infectées non traitées.

### Test antiviral dans les cellules MT2

Les cellules MT2 ont été concentrées à 3.10⁶/mL et infectées avec 10⁷ virus (LAI). Les cellules ont été réparties dans des plaques 96 puits (1.10⁵/puits) et incubées en présence de différentes concentrations du composé **A** (en quadriplicat). Les charges virales ont été réalisées au jour 3 (Test Cobas AmpliPrep/Cobas TaqMan (CTM) HIV-1 v2).

### Tests de cytotoxicité

Un test de viabilité cellulaire mesurant l'absorbance à 560 et 690 nm en utilisant le réactif jaune tétrazolium MTS [bromure de 3-(4,5-diméthyl-2-thiazolyl)-2,5-diphényltétrazolium] a été réalisé avec l'adénosine tétrabenzoylée dans les lignées cellulaires HeLa-CD4, Vero, FH, MT-2, et SupT1.

### Résultats

### Activité antivirale envers le VIH

L'activité antivirale du composé **A** selon l'invention a été évaluée en utilisant une quantité de VIH correspondant à 3 ng d'antigène p24. Pour comparaison, est présentée ici l'activité obtenue avec un composé ne présentant pas de double benzoylation en position N6.

| Structure | Production virale (10 µM de composé) |
|---|---|
| 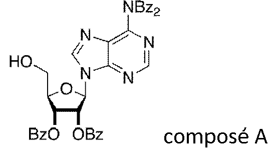 | 0% |
| 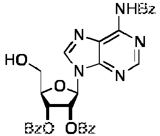 | 109% |

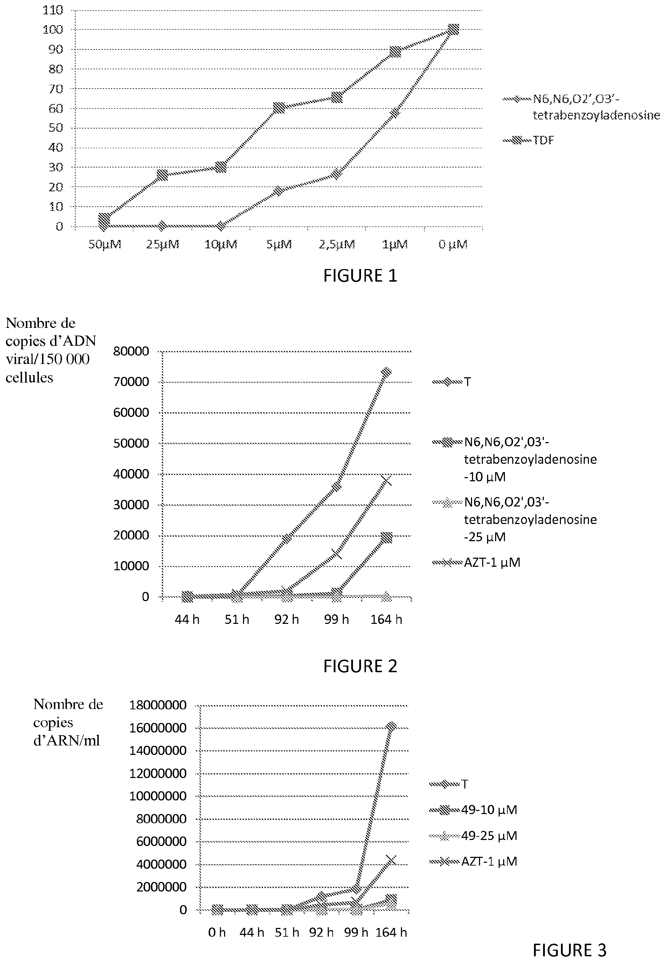

En utilisant une quantité de VIH correspondant à 3 ng d'antigène p24, l'IC50 de l'adénosine tétrabenzoylée (composé **A**) est entre 1 et 2 µM. Dans les mêmes conditions, l'IC50 du TDF (Ténofovir) est entre 5 et 10 µM (voir Figure 1).

Le composé **A** selon l'invention a été testé sur le virus pNL4-3 modifié ou non par mutagénèse sur site, comprenant ou non (wt) les mutations localisées dans le gène de la transcriptase inverse et associées avec la résistance aux principaux NRTIs utilisés en pratique clinique. Les tableaux 1A à 1C ci-dessous regroupent les index de résistance du composé A selon l'invention sur les virus avec et sans mutation.

L'index de résistance d'un mutant représente le rapport de la concentration en composé capable d'inhiber par moitié (IC50) la croissance du virus mutant testé sur la concentration en composé capable d'inhiber par moitié (IC50) la croissance du virus sauvage.

Un index de résistance inférieur ou égal à 1 signifie que la souche virale testée est sensible au traitement, ou en d'autres termes, que le composé inhibe efficacement la multiplication du virus. Un index de résistance supérieur à environ 1,5 ou 2 signifie que la souche virale est résistante au traitement.

**Tableau 1A: Index de résistance (« fold change ») des mutants aux inhibiteurs nucléosidiques de la transcriptase inverse.**

| | Index de résistance |
|---|---|
| pnL4-3 wild type | 1 |
| pNL4-3 T215Y | 0.41 |
| pNL4-3 M41L-L210W-T215Y | 0.38 |
| p N L4-3 M184V | 0.99 |
| pNL4-3 K65R | 0.92 |
| pNL4-3 K65R-M184V | 1,07 |
| pNL4-3 M184V-M41L-L210W-T215Y | 0,58 |

Le composé **A** a été testé sur des cellules MT2 infectées par des virus LAI. L'IC50 a été déterminée dans ce cas à 1,6µM.

**Tableau 1B : Index de résistance des mutants aux inhibiteurs non nucléosidiques de la transcriptase inverse**

| | Index de résistance |
|---|---|
| pnL4-3 wild type | 1 |
| pNL4-3 K103N | 1 |
| pNL4-3 E138K | 0,89 |

**Tableau 1C: Index de résistance des mutants aux inhibiteurs de l'intégrase**

| | Index de résistance |
|---|---|
| pnL4-3 wild type | 1 |
| pNL4-3 N155H | 1,04 |
| pNL4-3 G140S-Q184H | 0.99 |
| pNL4-3 Q184H | 0.88 |

Tous les mutants résistants sont sensibles au composé **A**, et même plus sensibles pour certains (index de résistance < 1) comme notamment ceux porteurs de la mutation T215Y.

### Essai de cytotoxicité

En utilisant différents types cellulaires (HeLa-CD4, Vero, FH, MT-2, et SupT1), la CC50 (concentration cytotoxique à 50%) du composé **A** a été mesurée à plus de 100 µM dans tous les cas.

### Exemple 3 : Inhibition de la production d'ADN proviral double brin et de production d'ARN cellulaire

Les cellules MT2 ont été concentrées à 15.10⁶/mL et incubées 2 h avec 10 ou 25 µM du composé A. Le virus Laï est ensuite ajouté à une multiplicité d'infection de 0,01 pendant 1 h. Les cellules sont lavées puis incubées dans du milieu de culture contenant 10 ou 25 µM du composé à tester. Un prélèvement de 1.10⁶ cellules est réalisé à différents temps et l'ADN viral est quantifié par une méthode de PCR en temps réel Taqman. Les charges virales ARN sont quantifiées dans le milieu de culture (Test Cobas AmpliPrep/Cobas TaqMan (CTM) HIV-1 v2).

Les résultats sont présentés aux Figures 2 et 3. Ils montrent que l'apparition de l'ADN viral intracellulaire (Figure 2), et la production de l'ARN viral extracellulaire (Figure 3) sont inhibées par le composé **A**. En d'autres termes, le composé **A** empêche la production d'ADN viral intracellulaire et par conséquent celle d'ARN extracellulaire. La comparaison avec l'AZT (zidovudine) montre la nette supériorité du composé **A** sur l'inhibition de la charge virale.

## Revendications

1. Composé de formule (I) 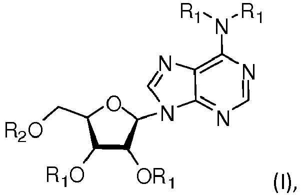 dans laquelle :
chaque R1 est indépendamment choisi parmi un groupement benzyle et un groupement benzoyle, éventuellement substitué par un ou plusieurs substituants choisis parmi un groupement nitro (NO₂) un groupement alkyle, et un atome d'halogène, et
R2 est choisi parmi un atome d'hydrogène, un groupement acyle, un monophosphate, diphosphate ou triphosphate, ou un dérivé phosphate stabilisé choisi parmi un groupe 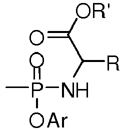 et un groupe 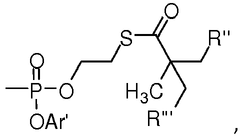 dans lesquels Ar et Ar' sont des groupements phényles ou naphtyles éventuellement substitués par un ou plusieurs atomes d'halogène, et R, R', R" et R'" sont indépendamment choisis parmi un atome d'hydrogène, un groupement hydroxy (OH), un alkyle, un phényle et un groupement amine NRaRb où Ra et Rb sont choisis parmi les atomes d'hydrogènes, les alkyles et les halogénoalkyles,
un tautomère ou énantiomère de celui-ci, ou un sel pharmaceutiquement acceptable de celui-ci, ou un mélange de ceux-ci,
pour une utilisation dans le traitement d'une infection virale.

2. Composé selon la revendication 1, pour une utilisation dans le traitement d'une infection virale, l'infection virale étant choisie parmi une infection par le virus VIH, le virus de l'hépatite C, le virus de l'hépatite B et le virus de l'herpès.

3. Composé selon la revendication 1 ou 2, pour une utilisation dans le traitement d'une infection virale, qui est une infection par le virus VIH.

4. Composé selon la revendication 3, pour une utilisation dans le traitement d'une infection virale, qui est une infection par le virus VIH-1.

5. Composé selon la revendication 3 ou 4, pour une utilisation dans le traitement de patients infectés par le virus VIH et résistants aux inhibiteurs de transcriptase inverse nucléosidiques.

6. Composé, pour une utilisation dans le traitement d'une infection virale selon l'une quelconque des revendications précédentes, dans lequel les quatre groupements R1 sont des groupements benzoyles.

7. Composé, pour une utilisation dans le traitement d'une infection virale selon l'une quelconque des revendications 1 à 6, dans lequel R2 est un atome d'hydrogène.

8. Composé, pour une utilisation dans le traitement d'une infection virale selon la revendication 7, dans lequel le composé est la N⁶,N⁶,O^{2'},O^{3'}-tétrabenzoyladénosine de formule A :

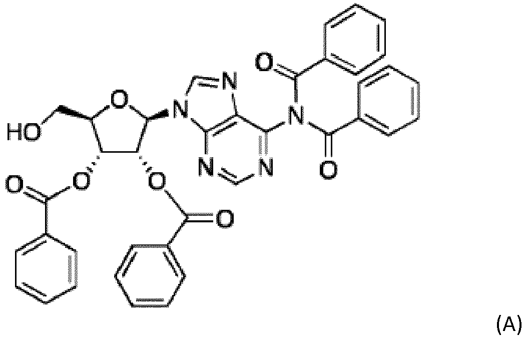

9. Composé tel que défini à l'une des revendications 1 à 8, à titre de médicament.

10. Composition pharmaceutique, comprenant au moins un composé tel que défini à l'une des revendications 1 à 8, et un support pharmaceutiquement acceptable.

11. Composition selon la revendication 10, dans laquelle le composé de formule (I) est la N⁶,N⁶,O^{2'},O^{3'}-tétrabenzoyladénosine de formule A

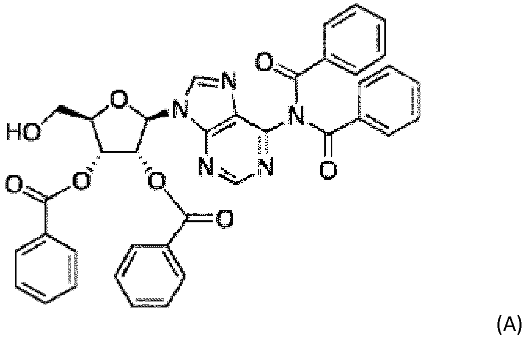

12. Composition pharmaceutique selon la revendication 10 ou 11, comprenant en outre un ou plusieurs autres antirétroviraux.

13. Composition pharmaceutique selon l'une quelconque des revendications 10 à 12, dans laquelle la composition est adaptée pour une administration par voie orale.

14. Méthode *in vitro* pour évaluer si un patient infecté par un virus VIH, notamment VIH-1, serait sensible à une thérapie avec un composé tel que défini à l'une des revendications 1 à 8, ladite méthode comprenant la recherche d'une mutation dans le codon 215 de la transcriptase inverse du virus, une substitution de la thréonine sauvage en tyrosine, étant indicatrice d'une plus grande sensibilité audit composé par rapport au virus sauvage.

## Patentansprüche

1. Verbindung der Formel (I): 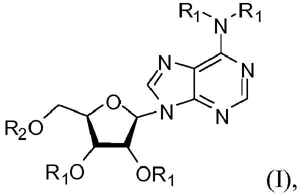 in der:
jeder R1 unabhängig voneinander aus einer Benzylgruppe und einer Benzoylgruppe ausgewählt ist, die gegebenenfalls mit einem oder mehreren Substituenten substituiert ist, die aus einer Nitrogruppe (NO₂), einer Alkylgruppe und einem Halogenatom ausgewählt sind, und
R2 aus einem Wasserstoffatom, einer Acylgruppe, einem Monophosphat, Diphosphat oder Triphosphat oder einem stabilisierten Phosphatderivat ausgewählt ist, das aus einer Gruppe 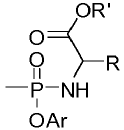 und einer Gruppe 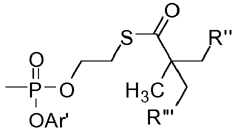 ausgewählt ist,
in denen Ar und Ar' Phenyl- oder Naphthylgruppen sind, die gegebenenfalls mit einem oder mehreren Halogenatomen substituiert sind, und R, R', R" und R'" unabhängig voneinander aus einem Wasserstoffatom, einer Hydroxygruppe (OH), einer Alkyl-, Phenyl- oder Aminogruppe NRaRb ausgewählt sind, wo Ra und Rb aus Wasserstoffatomen, Alkyl- und Halogenalkylgruppen ausgewählt sind,
ein Tautomer oder Enantiomer davon oder ein pharmazeutisch verträgliches Salz davon oder ein Gemisch davon,
für eine Verwendung in der Behandlung einer Virusinfektion.

2. Verbindung gemäß Anspruch 1 für eine Verwendung in der Behandlung einer Virusinfektion, wobei die Virusinfektion aus einer Infektion mit dem HI-Virus, Hepatitis C-Virus, Hepatitis B-Virus und dem Herpesvirus ausgewählt ist.

3. Verbindung gemäß Anspruch 1 oder 2 für eine Verwendung in der Behandlung einer Virusinfektion, die eine Infektion mit dem HI-Virus ist.

4. Verbindung gemäß Anspruch 3 für eine Verwendung in der Behandlung einer Virusinfektion, die eine Infektion mit dem Virus HIV-1 ist.

5. Verbindung gemäß Anspruch 3 oder 4 für eine Verwendung in der Behandlung von Patienten, die mit dem HI-Virus infiziert sind und gegen Nukleosid-Hemmer der reversen Transkriptase resistent sind.

6. Verbindung für eine Verwendung in der Behandlung einer Virusinfektion gemäß einem der vorhergehenden Ansprüche, wobei die vier R1-Gruppen Benzoylgruppen sind.

7. Verbindung für eine Verwendung in der Behandlung einer Virusinfektion gemäß einem der Ansprüche 1 bis 6, wobei R2 ein Wasserstoffatom ist.

8. Verbindung für eine Verwendung in der Behandlung einer Virusinfektion gemäß Anspruch 7, wobei die Verbindung N⁶,N⁶,O^{2'},O^{3'}-Tetrabenzoyladenosin der Formel A ist:

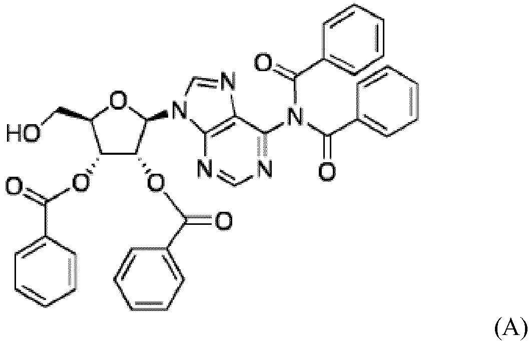

9. Verbindung, wie in einem der Ansprüche 1 bis 8 definiert, als Medikament.

10. Pharmazeutische Zusammensetzung, umfassend wenigstens eine wie in einem der Ansprüche 1 bis 8 definierte Verbindung und einen pharmazeutisch verträglichen Träger.

11. Zusammensetzung gemäß Anspruch 10, wobei die Verbindung der Formel (I) N⁶,N⁶,O^{2'},O^{3'}-Tetrabenzoyladenosin der Formel A ist

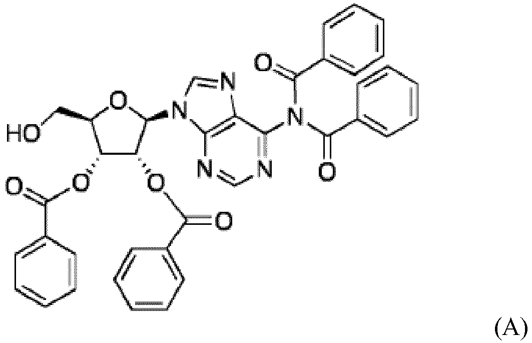

12. Pharmazeutische Zusammensetzung gemäß Anspruch 10 oder 11, umfassend außerdem eine oder mehrere andere antiretrovirale Verbindungen.

13. Pharmazeutische Zusammensetzung gemäß einem der Ansprüche 10 bis 12, wobei die Zusammensetzung für eine orale Verabreichung angepasst ist.

14. *In vitro* Verfahren zur Bewertung, ob ein Patient, der mit einem HI-Virus insbesondere mit HIV-1 infiziert ist, für eine Therapie mit einer wie in einem der Ansprüche 1 bis 8 definierten Verbindung empfindlich wäre, wobei besagtes Verfahren die Suche nach einer Mutation im Codon 215 der reversen Transkriptase des Virus umfasst, wobei eine Substitution von Wildtyp-Threonin durch Tyrosin ein Hinweis auf eine größere Empfindlichkeit für besagte Verbindung im Vergleich zum Wildtyp-Virus ist.

## Claims

1. Compound of formula (I) : 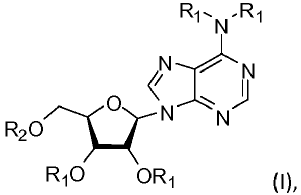 wherein:
each R1 is independently selected from a benzyl group and a benzoyl group, optionally substituted with one or more substituents chosen from a nitro group (NO₂), an alkyl group and halogen, and
R2 is selected from hydrogen, an acyl group, a monophosphate, diphosphate or triphosphate, or a stabilized phosphate derivative selected from a 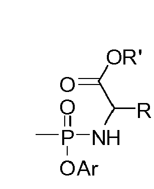 group and a 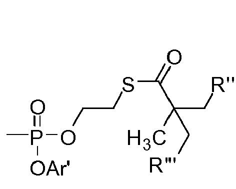 group, wherein Ar and Ar' are phenyl or naphthyl groups optionally substituted with one or more halogen atoms, and R, R', R" and R'" are independently selected from a hydrogen atom, a hydroxyl group (OH), an alkyl, a phenyl, and a NRaRb amine group, wherein Ra and Rb are selected from hydrogen atoms, alkyls and haloalkyls,
a tautomer or enantiomer thereof, or a pharmaceutically acceptable salt thereof, or a mixture thereof,
for use in the treatment of a viral infection.

2. Compound according to Claim 1, for use in the treatment of a viral infection, the viral infection being selected from an infection with HIV, hepatitis C virus, hepatitis B virus and herpes virus.

3. Compound according to Claim 1 or 2, for use in the treatment of a viral infection, which is an infection with HIV.

4. Compound according to Claim 3, for use in the treatment of a viral infection, which is an infection with HIV-1 virus.

5. Compound according to Claim 3 or 4, for use in the treatment of patients infected with the HIV virus and resistant to nucleoside reverse transcriptase inhibitors.

6. Compound for use in the treatment of a viral infection according to any one of the preceding claims, wherein the four R1 groups are benzoyl groups.

7. Compound for use in the treatment of a viral infection according to any one of Claims 1 to 6, wherein R2 is a hydrogen atom.

8. Compound for use in the treatment of a viral infection according to Claim 7, wherein the compound is N⁶,N⁶,O^{2'},O^{3'}-tetrabenzoyladenosine of formula A :

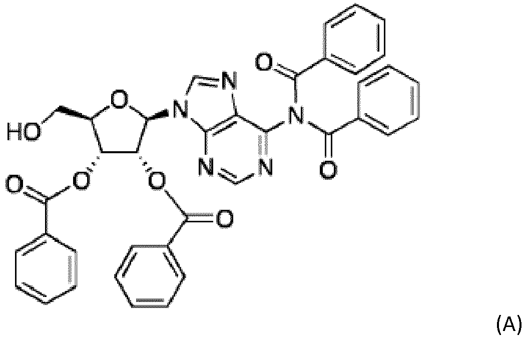

9. Compound as defined in one of Claims 1 to 8, as a medicament.

10. Pharmaceutical composition, comprising at least one compound as defined in one of Claims 1 to 8, and a pharmaceutically acceptable carrier.

11. Composition according to Claim 10, wherein the compound of formula (I) is N⁶,N⁶,O²,O^{3'}-tetrabenzoyladenosine of formula A

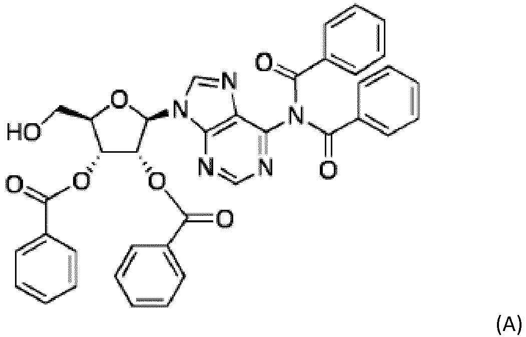

12. Pharmaceutical composition according to Claim 10 or 11, further comprising one or more other antiretrovirals.

13. Pharmaceutical composition according to any one of Claims 10 to 12, wherein the composition is suitable for oral administration.

14. An *in vitro* method for assessing whether a patient infected with a HIV virus, in particular HIV-1, would be sensitive to a therapy with a compound as defined in one of Claims 1 to 8, said method comprising searching for a mutation in codon 215 of the reverse transcriptase of the virus, a substitution of the wild-type threonine to tyrosine being indicative of a greater sensitivity to said compound compared with the wild-type virus.
